Europäisches Patentamt
European Patent Office
Office européen des brevets

(19)

(11) Publication number: 0 147 510 B1

# (12) EUROPEAN PATENT SPECIFICATION

(45) Date of publication of patent specification: **16.08.89**

(21) Application number: **84106699.6**

(22) Date of filing: **12.06.84**

(51) Int. Cl.$^4$: **C 07 D 311/80,** C 07 D 405/12 // A61K31/35, A61K31/395 ,(C07D405/12, 311:80, 233:60)

## (54) Dibenzopyran derivatives.

(30) Priority: **19.09.83 US 533475**

(43) Date of publication of application:
**10.07.85 Bulletin 85/28**

(45) Publication of the grant of the patent:
**16.08.89 Bulletin 89/33**

(84) Designated Contracting States:
**AT BE CH DE FR GB IT LI LU NL SE**

(56) References cited:
**GB-A-1 251 873**
**US-A-4 120 873**

(73) Proprietor: **PENNWALT CORPORATION**
**Pennwalt Building Three Parkway**
**Philadelphia Pennsylvania 19102 (US)**

(72) Inventor: **Griffith, Ronald Conrad**
**41 Northfield Gate**
**Pittsford, N.Y. (US)**

(74) Representative: **Kraus, Walter, Dr. et al**
**Patentanwälte Kraus, Weisert & Partner**
**Thomas-Wimmer-Ring 15**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates generally to dibenzopyran derivatives and more specifically to certain 7,8,9,10-tetrahydro-6H-dibenzo[b,d]pyranyloxyaminopropanols.

The compounds of this invention possess useful antihypoxia activity, that is they protect warm-blooded animals from the effects of oxygen deprivation.

US—A—4,120,873 describes benzopyrane compounds useful as analgesic agents and tranquilizers. GB—A—1,251,873 describes chromane compounds enhancing the blood flow through coronary vessels and influencing the activity of the adrenergic system in the alpha-adrenalytic sense.

In accordance with this invention, there are provided 7,8,9,10-tetrahydro-6H-dibenzo[b,d]pyranyloxy-aminopropanols having the following general structure (1):

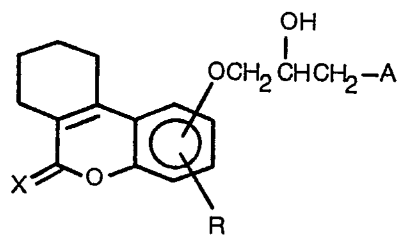

1

where X is oxygen or dialkyl in which each alkyl group contains one to seven carbons ($C_1$—$C_7$); R is hydrogen or alkyl containing one to seven carbons ($C_1$—$C_7$); A is

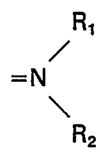

and where $R_1$ and $R_2$ may independently be hydrogen, $C_1$—$C_7$ alkyl, $C_3$—$C_7$ cycloalkyl, aryl, heteroaryl, $C_7$—$C_9$ aralkyl, aryloxyalkyl, $C_2$—$C_6$ alkanol, or where $R_1$ and $R_2$ taken together comprise a heterocyclic ring containing 1—3 heteroatoms and 2—7 carbon atoms.

This invention also includes acid addition salts of these compounds and the method of preparing the compounds.

The compounds of the invention can be conveniently prepared from 3-hydroxy and 1-hydroxy-3-methyl-7,8,9,10-tetrahydro-6H-dibenzo[b,d]pyrone and -pyrans which are known compounds. The pyrone and pyran starting materials can be prepared as described in R. Adams and B. R. Baker, *J. Am. Chem. Soc.*, *62,* 2405 (1940). Thus, condensing orcinol or resorcinol, with ethyl cyclohexanone-2-carboxylate in the presence of phosphorus oxychloride, gives the corresponding pyrones, which are converted to the 6,6-dimethyl pyrans (formula 2) upon treatment with methyl magnesium iodide.

Treatment of these phenolic pyrans (2) with an alkali hydroxide such as sodium or potassium hydroxide, and an excess of an epihalohydrin, such as epichlorohydrin in a suitable inert solvent, gives the corresponding epoxides *(3),* where X and R are as described for *(1).*

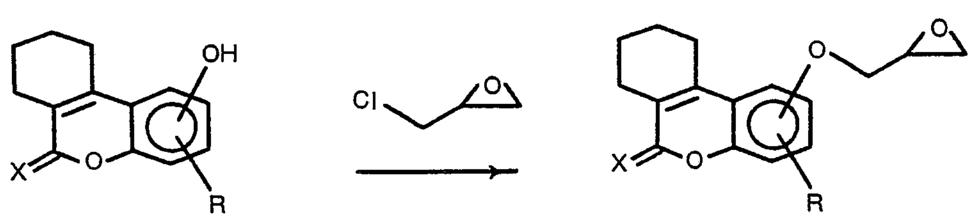

2 3

The compounds of general formula *1,* where A is primary secondary or tertiary amino, are prepared from the epoxides (formula 3) by treatment with a solution of ammonia or the corresponding primary or secondary amine in a suitable solvent (e.g. methanol, chloroform, tetrahydrofuran).

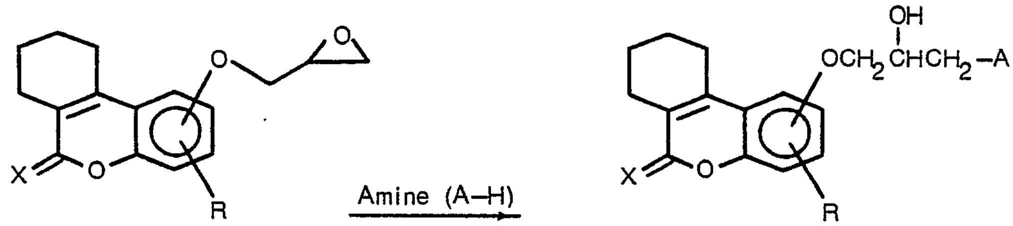


3 1

The compounds of the invention also include acid addition salts which are pharmaceutically acceptable. Acid addition salts include those derived from both organic and inorganic acids such as, for example, acetic acid, maleic acid, malic acid, fumaric acid, succinic acid, succinamic acid, tartaric acid, citric acid, lactic acid, hydrochloric acid, hydrobromic acid, hydriodic acid, sulfuric acid, phosphoric acid, and the like.

Amines which can be reacted to form the compounds of the invention A—H where

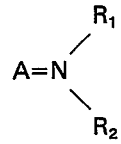

and where $R_1$ and $R_2$ may independently be hydrogen, $C_1$—$C_7$ alkyl, $C_3$—$C_7$ cycloalkyl, aryl, heteroaryl, $C_7$—$C_9$ aralkyl, aryloxyalkyl, $C_2$—$C_6$ alkanol, or where $R_1$ and $R_2$ taken together comprise a heterocyclic ring containing 1—3 heteroatoms and 2—7 carbon atoms.

The following specific examples are provided to illustrate the preparation of the epoxides (3) and their conversion to the compounds of general formula (1) by treatment with a variety of amines, including ammonia, methylamine, dimethylamine, ethylamine, diethylamine, n-propylamine, isopropylamine, cyclopropylamine, t-butylamine, ethanolamine, cyclopentylamine, pyrrolidine, piperidine, aniline, benzylamine, imidazole, 1-phenoxy-2-propylamine, and 3-amino-6-oxo-6H-dibenzo[b,d]pyran.

## Preparation of Intermediates

### Procedure 1

Preparation of 7,8,9,10-tetrahydro-3-methyl-1-(1-propoxy-2,3-oxide)-6-oxo-6H-dibenzo[b,d]pyran

To a stirred solution of NaOH (4.0 g, 0.1 m) in dimethylsulfoxide (DMSO) (250 ml) and water (250 ml), was added 7,8,9,10-tetrahydro-1-hydroxy-3-methyl-6-oxo-6H-dibenzo[b,d]pyran (20 g, 0.087 m) and the mixture stirred for 5 minutes, then treated with epichlorohydrin (50 ml). After 5 hours the mixture was cooled in an ice bath for 30 minutes and the precipitated solid collected by filtration, washed with water and dried to give 7,8,9,10-tetrahydro-3-methyl-1-(1-propoxy-2,3-oxide)-6-oxo-6H-dibenzo[b,d]pyran as a white solid, mp. 164—165°C. An analytical sample recrsytallized from methanol melted at 165—166°C,

### Procedure 2

Preparation of 7,8,9,10-tetrahydro-3-(1-propoxy-2,3-oxide)-6-oxo-6H-dibenzo[b,d]pyran

To a stirred solution of NaOH (2.0 g, 0.05 m) in DMSO (125 ml) and water (125 ml) was added 7,8,9,10-tetrahydro-3-hydroxy-6-oxo-6H-dibenzo[b,d]pyran (10.0 g, 0.046 m). When a solution was obtained, the mixture was treated with epichlorohydrin (35 ml) and stirred for 56 hours. A white solid precipitated which was collected by filtration to give 12.1 g of a mixture consisting of ca. 90% 7,8,9,10-tetrahydro-3-(1-propoxy-2,3-oxide)-6-oxo-6H-dibenzo[b,d]pyranyl-3-oxy)-2-propanol. The pure epoxide was obtained by chromatography on $SiO_2$ and crystallization from methanol/chloroform to give 8.0 g of white solid, mp. 120—121°C.

### Procedure 3

Preparation of 7,8,9,10-tetrahydro-3,6,6-trimethyl-1-(1-propoxy-2,3-oxide)-6H-dibenzo[b,d]pyran

To a stirred solution of NaOH (2.4 g, 0.06 m) in DMSO (125 ml) and water (125 ml) was added 7,8,9,10-tetrahydro-3,6,6-trimethyl-1-hydroxy-6H-dibenzo[b,d]pyran (13.1 g, 0.054 m) in DMSO (125 ml) and water (125 ml) and then epichlorohydrin (34.8 g, 0.376 m) and the mixture stirred for 5 hours, treated with water (0.5 L) and extracted with ether (3 × 300 ml). The combined ether extracts were dried over $MgSO_4$ and evaporated to give 7,8,9,10-tetrahydro-3,6,6-trimethyl-1-(1-propoxy-2,3-oxide)-6H-dibenzo[b,d]pyran as a pale yellow oil, 14.0 g. This material was of sufficient purity to use as is for further chemical processing.

### Procedure 4

Preparation of 7,8,9,10-tetrahydro-6,6-dimethyl-3-(1-propoxy-2,3-oxide)-6H-dibenzo[b,d]pyran

To a stirred solution of NaOH (4.0 g, 0.1 m) DMSO (250 ml) and water (250 ml) was added 7,8,9,10-tetrahydro-6,6-dimethyl-3-hydroxy-6H-dibenzo[b,d]pyran (21.3 g, 0.0926 m) and then epichlorohydrin (60 g, 0.648 m) and the mixture stirred for 5 hours, treated with water (1 L) and extracted with ether (3 × 500 ml). The combined extracts were dried over $MgSO_4$ and evaporated to give 7,8,9,10-tetrahydro-6,6-dimethyl-3-(1-propoxy-2,3-oxide)-6H-dibenzo[b,d]pyran as a pale yellow oil, 23.2 g. This material was of sufficient purity to use as is for further chemical processing.

Example 1

Preparation of 3-(7,8,9,10-tetrahydro-6-oxo-6H-dibenzo[b,d]pyranyl-3-oxy)-1-(1-methylethyl)amino-2-propanol hydrochloride.

To a stirred solution of 7,8,9,10-tetrahydro-3-(1-propoxy-2,3-oxide)-6-oxo-6H-dibenzo[b,d]pyran (11.6 g, 0.0426 m) in methanol (250 ml) was added isopropyl amine (25 ml) and the mixture heated to reflux for 1 hour, cooled; and the solvent evaporated. The residue was dissolved in chloroform (200 ml) and 5% HCl (250 ml), the layers separated and the aqueous phase extracted with (2 × 100 ml) chloroform. The combined chloroform extracts were backwashed with 100 ml 5% HCl and the combined aqueous acid layers were basified to pH 11 with NaOH, and extracted with chloroform (3 × 200 ml). These extracts were dried and evaporated to a white solid 11.8 g. This was dissolved in methanol/isopropanol, acidified with HCl gas and the salt allowed to crystallize. The white solid was collected by filtration, recrystallized a second time and vacuum dried to give 9.5 g of 3-(7,8,9,10-tetrahydro-6-oxo-6H-dibenzo[b,d]pyranyl-3-oxy)-1-(1-methylethyl)amino-2-propanol hydrochloride, mp. 205—206°C.

Example 2

Preparation of 3-(7,8,9,10-tetrahydro-3-methyl-6-oxo-6H-dibenzo[b,d]pyranyl-1-oxy)-1-(1-methylethyl)-amino-2-propanol hydrochloride

A solution of 7,8,9,10-tetrahydro-3-methyl-1-(1-propoxy-2,3-oxide)-6-oxo-6H-dibenzo[b,d]pyran (12 g, 0.042 m) in methanol (250 ml) was treated with isopropylamine (25 ml) and the mixture stirred and heated to reflux for 1 hour, cooled, and the solvent evaporated to an oil that solidified on standing. This was dissolved in methanol (50 ml) and isopropanol (50 ml), acidified with HCl gas and the salt allowed to crystallize. The white solid was collected by filtration and vacuum dried to give 8.4 g of 3 - (7,8,9,10 - tetrahydro - 3 - methyl - 6 - oxo - 6H - dibenzo[b,d]pyranyl - 1 - oxy) - 1 - (1 - methylethyl)amino - 2 - propanol hydrochloride, mp. 259—260°C.

Example 3

Preparation of 3-(7,8,9,10-tetrahydro-3-methyl-6-oxo-6H-dibenzo[b,d]pyranyl-1-oxy)-1-(1,1-dimethylethyl)-amino-2-propanol hydrochloride

A solution of 7,8,9,10-tetrahydro-3-methyl-1-(1-propoxy-2,3-oxide)-6-oxo-6H-dibenzo[b,d]pyran (9.4 g, 0.033 m) in methanol (250 ml) was treated with t-butylamine and the mixture stirred and heated to reflux for 1 hour, cooled, and the solvent evaporated to an off-white solid. This material was purified by chromatography on a Prep 500 HPLC, on silica gel, eluting with chloroform and ammoniated methanol to give a white solid, 11.5 g. This was dissolved in methanol (150 ml) and isopropanol and acidified with HCl gas. Upon standing a white solid crystallized which was collected by filtration and vacuum dried to give 12.0 g of 3 - (7,8,9,10 - tetrahydro - 3 - methyl - 6 - oxo - 6H - dibenzo[b,d]pyranyl - 1 - oxy - 1 - (1,1 - dimethylethyl)amino - 2 - propanol hydrochloride, mp. 304—305°C.

Example 4

Preparation of 3-(7,8,9,10-tetrahydro-3-methyl-6-oxo-6H-dibenzo[b,d]pyranyl-1-oxy)-1-(1-methyl-2-phenoxy ethyl)amino-2-propanol hydrochloride

To a stirred solution of 7,8,9,10-tetrahydro-3-methyl-1-(1-propoxy-2,3-oxide)-6-oxo-6H-dibenzo[b,d]-pyran (11.8 g, 0.041 m) in methanol (250 ml) was added 1-methyl-2-phenoxyethylamine (15 g, 0.1 m) and the mixture refluxed for 5 hours, cooled, and the solvent evaporated. The residue was dissolved in chloroform (2 × 50 ml) and the combined chloroform extracts dried and evaporated to a foamy solid residue. This was recrsytallized twice from a mixture of ethanol, chloroform, and ether and vacuum dried to give 9.1 g of 3 - (7,8,9,10 - tetrahydro - 3 - methyl - 6 - oxo - 6H - dibenzo[b,d]pyranyl - 1 - oxy) - 1 - (1 - methyl - 2 - phenoxy ethyl)amino - 1 - propanol hydrochloride as a white solid, mp. 202—203°C.

Example 5

Preparation of 3-(7,8,9,10-tetrahydro-6,6-dimethyl-6H-dibenzo[b,d]pyranyl-3-oxy)-1-(1-methylethyl)amino-2-propanol hydrochloride

To a stirred solution of 7,8,9,10-tetrahydro-6,6-dimethyl-3-(1-propoxy-2,3-oxide)-6H-dibenzo[b,d]pyran in methanol (500 ml) was added isopropylamine (50 ml) and the mixture heated to reflux for 1 hour, cooled and the solvent evaporated to a solid residue. This was dissolved in isopropanol (50 ml) and ether (350 ml) and acidified with HCl gas. On cooling, a white solid crystallized, which was recrsytallized twice and vacuum dried to give 9.1 g of 3 - (7,8,9,10 - tetrahydro - 6,6 - dimethyl - 6H - dibenzo[b,d]pyranyl - 3 - oxy) - 1 - (1 - methylethyl)amino - 2 - propanol hydrochloride, mp 184—186°C.

Example 6

Preparation of 3-(7,8,9,10-tetrahydro-3,6,6-trimethyl-6H-dibenzo[b,d]pyranyl-1-oxy)-1-(1-methylethyl)-amino-2-propanol hydrochloride

To a stirred solution of 7,8,9,10-tetrahydro-3,6,6-trimethyl-1-(1-propoxy-2,3-oxide)-6H-dibenzo[b,d]-pyran (14 g, 0.046 m) in methanol (250 ml) was added isopropylamine (30 ml) and the mixture heated to reflux for 1 hour, cooled, and the solvent evaporated. The residue was purified by chromatography on a Prep 500 HPLC, on silica gel, eluting with chloroform and ammoniated methanol, to give a yellow oil. This was dissolved in isopropanol (200 ml) and ether (300 ml), acidified with HCl gas. Upon standing, a white solid crystallized which was collected by filtration and vacuum dried to give 8.7 g of 3 - (7,8,9,10 - tetrahydro - 3,6,6 - trimethyl - 6H - dibenzo[b,d]pyranyl - 1 - oxy) - 1 - (1 - methylethyl)amino - 2 - propanol hydrochloride, mp 180—182°C.

Example 7

Preparation of 3-(7,8,9,10-tetrahydro-3,6,6-trimethyl-6H-dibenzo[b,d]pyranyl-1-oxy)-1-(1,1-dimethylethyl)-amino-2-propanol hydrochloride

To a stirred solution of 7,8,9,10-tetrahydro-3,6,6-trimethyl-1-(1-propoxy-2,3-oxide)-6H-dibenzo[b,d]-pyran (18 g, 0.06 m) in methanol (250 ml) was added t-butylamine (30 ml) and the mixture heated to reflux for 1 hour, cooled, and the solvent evaporated to an oil. The oil was dissolved in isopropanol (100 ml) and ether (300 ml), hot filtered, and acidified with HCl gas. Upon addition of ether (100 ml) and cooling, a solid crystallized which was collected by filtration, recrystallized as above and dried under vacuum to give 6.0 g of 3 - (7,8,9,10 - tetrahydro - 3,6,6 - trimethyl - 6H - dibenzo[b,d]pyranyl - 1 - oxy) - 1 - (1,1 - dimethylethyl)amino - 2 - propanol hydrochloride as a white solid, mp. 158—160°C.

Example 8

Preparation of 3-(7,8,9,10-tetrahydro-3,6,6-trimethyl-6H-dibenzo[b,d]pyranyl-1-oxy)-1-methylamino-2-propanol hydrochloride

To a stirred solution of 7,8,9,10-tetrahydro-3,6,6-trimethyl-1-(1-propoxy-2,3-oxide)-6H-dibenzo[b,d]-pyran (12.4 g, 0.041 m) in methanol (500 ml) was added methylamine (50 ml) and the mixture stirred to 25°C for 4 hours, then 50°C for 1 hour. The mixture was allowed to cool and the solvent evaporated to an oil, 14.2 g. This was purified by chromatography on a Prep 500 HPLC on silica gel, eulting with ammoniated methanol and chloroform to give an oil, 9.8 g. This was dissolved in isopropanol (50 ml) and ether (200 ml) and acidified with HCl gas. Upon standing, a white solid crystallized, which was collected, recrystallized and vacuum dried to give 5.4 g of 3 - (7,8,9,10 - tetrahydro - 3,6,6 - trimethyl - 6H - dibenzo[b,d]pyranyl - 1 - oxy) - 1 - methylamino - 2 - propanol hydrochloride, mp. 187—188°C.

Example 9

Preparation of 3-(7,8,9,10-tetrahydro-6-oxo-6H-dibenzo[b,d]pyranyl-3-oxy)-1-(1-piperidinyl)-2-propanol hydrochloride

To a stirred solution of 7,8,9,10-tetrahydro-3-(1-propoxy-2,3-oxide)-6-oxo-6H-dibenzo[b,d]pyran (10.0 g, 0.036 m) is methanol (250 ml) and chloroform (50 ml) under an atmosphere of nitrogen was added piperidine (3.46 g, 0.04 m) and the mixture stirred 18 hours. The solvent was evaporated to a residue, 13.4 g, which was purified by chromatography on a Prep 500 HPLC, on silica gel, eluting with chloroform and 5% ammoniated methanol to give an oil. This was dissolved in isopropanol (100 ml) and methanol (100 ml) and acidified with HCl gas. Upon standing, a white solid crystallized which was collected by filtration and vacuum dried to give 4.0 g of 3 - (7,8,9,10 - tetrahydro - 6 - oxo - 6H - dibenzo[b,d]pyranyl - 3 - oxy) - 1 - (1 - piperidinyl) - 2 - propanol hydrochloride, mp. 226—227°C.

Example 10

Preparation of 3-(7,8,9,10-tetrahydro-6-oxo-6H-dibenzo[b,d]pyranyl-3-oxy)-1-ethylamino-2-propanol hydrochloride

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added monoethylamine (25 mls) and the mixture stirred 48 hours. The solvent was evaporated to a residue, 13.4 g, which was purified by chromatography on a Prep 500 HPLC, on silica gel, eluting with chloroform and 5% ammoniated methanol to give a solid residue. This was dissolved in absolute ethanol (150 ml) and acidified with HCl gas. Upon standing, a white solid crystallized which was collected by filtration and vacuum dried to give 10.6 g of 3-(7,8,9,10-tetrahydro-6-oxo-6H-dibenzo[b,d]pyranyl-3-oxy)-1-ethylamino-2-propanol hydrochloride, mp. 193—194°C.

Example 11

Preparation of 3-(7,8,9,10-tetrahydro-6-oxo-6H-dibenzo[b,d]pyranyl-1-oxy)-1-methylamino-2-propanol hydrochloride

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added monomethylamine (25 ml) and the mixture stirred 18 hours. The solvent was evaporated to a residue, 13.6

g, which was purified by chromatography on a Prep 500 HPLC, on silica gel, eluting with chloroform and 5% ammoniated methanol, to give a white solid. This was dissolved in absolute ethanol (200 ml) and isopropanol (200 ml), acidified with HCl gas. Upon standing, a white solid crystallized which was collected by filtration and vacuum dried to give 6.9 g of 3-(7,8,9,10-tetrahydro-6-oxo-6H-dibenzo[b,d]pyranyl-3-oxy)-1-methylamino-2-propanol hydrochloride, mp. 218—219°C.

Example 12

Preparation of 3-(7,8,9,10-tetrahydro-6-oxo-6H-dibenzo[b,d]pyranyl-3-oxy)-1-dimethylamino-2-propanol hydrochloride

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added dimethylamine (25 ml) and the mixture stirred 18 hours. The solvent was evaporated to an oily residue, 14.2 g. This was purified by chromatography on a Prep 500 HPLC, on silica gel, eluting with chloroform and 5% ammoniated methanol, to give a white solid. This was dissolved in isopropanol (100 ml) and methanol (100 ml) and acidified with HCl gas. Upon standing, a solid crystallized which was collected by filtration and vacuum dried to give 6.4 g of 3-(7,8,9,10-tetrahydro-6-oxo-6H-dibenzo[b,d]pyranyl-3-oxy)-1-dimethylamino-2-propanol hydrochloride, mp. 205—206°C.

Example 13

Preparation of 3-(7,8,9,10-tetrahydro-6-oxo-6H-dibenzo[b,d]pyranyl-3-oxy)-1-(propylamino)-2-propanol hydrochloride

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added n-propylamine (2.4 g; 0.041 m) and the mixture was stirred for 72 hours. The solvent was evaporated to an oily residue, 13.1 g, which was purified by chromatography on a Prep 500 HPLC, on silica gel, eluting with chloroform and 5% ammoniated methanol, to give 6.1 g of a white solid. This was dissolved in methanol (100 ml) and isopropanol (100 ml) and acidified with HCl gas. Upon standing, a white solid crystallized which was collected by filtration and vacuum dried to give 4.2 g of 3-(7,8,9,10-tetrahydro-6-oxo-6H-dibenzo-[b,d]pyranyl-3-oxy)-1-(propylamino)-2-propanol hydrochloride, mp. 203—204°C.

Example 14

Preparation of 3-(7,8,9,10-tetrahydro-6-oxo-6H-dibenzo[b,d]pyranyl-3-oxy)-1-(diethylamino)-2-propanol hydrochloride

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added diethylamine (2.9 g, 0.04 m) and the mixture stirred 24 hours. The reaction was recharged with diethylamine (2.9 g, 0.04 m) and heated to 35°C for 24 hours. The solvent was evaporated to give an oil, 13.4 g, which was purified by chromatography on a Prep 500 HPLC, on silica gel, eluting with chloroform and 5% ammoniated methanol, to give an oil, 10.6 g. This was dissolved in methanol (100 ml) and isopropanol (100 ml) and acidified with HCl gas. Upon standing, a white solid crystallized which was collected by filtration and vacuum dried to give 8.2 grams of 3-(7,8,9,10-tetrahydro-6-oxo-6H-dibenzo[b,d]pyranyl-3-oxy)-1-(diethylamino)-2-propanol hydrochloride, mp. 149—150°C.

Example 15

Preparation of 3-(7,8,9,10-tetrahydro-6-oxo-6H-dibenzo[b,d]pyranyl-3-oxy)-1-(1-methyl-2-phenoxyethyl)-amino-2-propanol hydrochloride

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added 1-methyl-2-phenylethylamine (6.0 g, 0.04 m) and the mixture stirred for 96 hours. The solvent was evaporated to an oily residue, 14.4 g, which was purified by chromatography on a Prep 500 HPLC, on silica gel, eluting with chloroform and 5% ammoniated methanol to give 4.4 g of the pure amine base. This was dissolved in absolute ethanol (100 ml) and acidified with HCl gas. Upon standing, a white solid crystallized which was collected by filtration and vacuum dried to give 4.0 g of 3 - (7,8,9,10 - tetrahydro - 6 - oxo - 6H - dibenzo[b,d]pyranyl - 3 - oxy) - 1 - (1 - methyl - 2 - phenoxyethyl)amino - 2 - propanol hydrochloride, m.p. 178—179°C.

Example 16

Preparation of 3-(7,8,9,10-tetrahydro-6-oxo-6H-dibenzo[b,d]pyranyl-3-oxy)-1-(1-pyrrolidinyl)-2-propanol hydrochloride

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added pyrrolidine (3.4 g, 0.05 m) and the mixture stirred 18 hours. The solvent was evaporated to a residue, 13.4 g, which was purified by chromatography on a Prep 500 HPLC, on silica gel, eluting with chloroform and 5% ammoniated methanol to give the pure amine base as an oil. This was dissolved in methanol (50 ml) and isopropanol (50 ml) and acidified with HCl gas. Upon standing, a white solid crystallized which was

collected by filtration and vacuum dried to give 7.3 g of 3 - (7,8,9,10 - tetrahydro - 6 - oxo - 6H - dibenzo-[b,d]pyranyl - 3 - oxy) - 1 - (1 - pyrrolidinyl) - 2 - propanol hydrochloride, mp 195—196°C.

Example 17

Preparation of 3-(7,8,9,10-tetrahydro-6-oxo-6H-dibenzo[b,d]pyranyl-3-oxy)-1-(phenylmethyl)amino-2-propanol hydrochloride

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added benzylamine (4.3 g, 0.04 m) and the mixture stirred 48 hours. The solvent was evaporated to a residue, 13.1 g, which was purified by chromatography on a Prep 500 HPLC, on silica gel, eluting with chloroform and 5% ammoniated methanol, to give the pure free base as an oil. This was dissolved in methanol (100 ml) and isopropanol (100 ml) and acidified with HCl gas. Upon standing, a white solid crystallized which was collected by filtration and vacuum dried to give 5.7 g 3 - (7,8,9,10 - tetrahydro - 6 - oxo - 6H - dibenzo-[b,d]pyranyl - 3 - oxy) - 1 - (phenylmethyl)amino - 2 - propanol hydrochloride, mp. 222—223°C.

Example 18

Preparation of 3-(7,8,9,10-tetrahydro-6-oxo-6H-dibenzo[b,d]pyranyl-3-oxy)-1-(cyclopropylamino)-2-propanol hydrochloride

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added cyclopropylamine (5.7 g, 0.10 m) and the mixture stirred for 18 hours. The solvent was evaporated to an oily residue, 12.6 g, which was purified by chromatography on a Prep 500 HPLC, on silica gel, eluting with chloroform and 5% ammoniated methanol, to give the pure amine base as a solid, 6.4 g. This was dissolved methanol (150 ml) and isopropanol (150 ml) and acidified with HCl gas. Upon standing, a white solid crystallized which was collected by filtration and vacuum dried to give 5.7 g of 3 - (7,8,9,10 - tetrahydro - 6 - oxo - 6H - dibenzo[b,d]pyranyl - 3 - oxy) - 1 - (cyclopropylamino) - 2 - propanol hydrochloride, mp 185—186°C.

Example 19

Preparation of 3-(7,8,9,10-tetrahydro-6-oxo-6H-dibenzo[b,d]pyranyl-3-oxy)-1-(1,1-dimethylethyl)amino-2-propanol hydrochloride

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added t-butylamine (5.2 g, 0.072 m) and the mixture stirred for 18 hours. The solvent was evaporated to a solid residue, 13.5 g, which was purified by chromatography on a Prep 500 HPLC, on silica gel, eluting with chloroform and 5% ammoniated methanol to give the pure amine as a solid. This was dissolved in methanol (150 ml) and isopropanol (150 ml) and acidified with HCl gas. Upon standing, a white solid crystallized which was collected by filtration and vacuum dried to give 10.6 g 3 - (7,8,9,10 - tetrahydro - 6 - oxo - 6H - dibenzo[b,d]pyranyl - 3 - oxy) - 1 - (1,1 - dimethylethyl)amino - 2 - propanol hydrochloride, mp. 286—287°C.

Example 20

Preparation of 3-(7,8,9,10-tetrahydro-6-oxo-6H-dibenzo[b,d]pyranyl-3-oxy)-1-(2-hydroxyethyl)amino-2-propanol

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added ethanolamine (4.3 g, 0.072 m) and the mxiture stirred for 48 hours. The solvent was evaporated to a residue, 13.4 g, which was purified by chromatography on a Prep 500 HPLC, on silica gel, eluting with toluene and 10% ammoniated isopropanol to give the pure amine as an oil, 8.4 g. This was dissolved in hot absolute ethanol (75 ml) and upon standing, a white solid crystallized which was collected by filtration and vacuum dried to give 5.2 g of 7,8,9,10 - tetrahydro - 6 - oxo - 6H - dibenzo[b,d]pyranyl - 3 - oxy) - 1 - (2 - hydroxyethyl)amino - 2 - propanol, mp. 79—80°C.

Example 21

Preparation of 3-(7,8,9,10-tetrahydro-6-oxo-6H-dibenzo[b,d]pyranyl-3-oxy)-1-(phenylamino)-2-propanol hydrochloride

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added aniline (6.2 g, 0.074 m) and the mixture heated to reflux for 1 hour. The solvent was evaporated to a residue, 13.4 g, which was purified by chromatography on a Prep 500 HPLC, on silica gel, eluting with chloroform and 5% ammoniated methanol, to give the amine base as an oil. This was dissolved in absolute ethanol (100 ml) and ethylacetate (100 ml) and acidified with HCl gas. Upon standing, a white solid crystallized which was collected by filtration and vacuum dried to give 3.1 g of 3 - (7,8,9,10 - tetrahydro - 6 - oxo - 6H - dibenzo[b,d]pyranyl - 3 - oxy) - 1 - (phenylamino)-2-propanol hydrochloride, mp. 173—174°C.

Example 22

Preparation of 3-(7,8,9,10-tetrahydro-6-oxo-6H-dibenzo[b,d]pyranyl-3-oxy)-1-cyclopentylamino-2-propanol hydrochloride

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added cyclopentylamine (6.3 g, 0.074 m) and the mixture heated to reflux for 1 hour. The solvent was evaporated to an oily residue, which was purified by chromatography on a Prep 500 HPLC, on silica gel, eluting with chloroform and 3% ammoniated methanol to give the pure amine base as a solid. This was dissolved in absolute ethanol (200 ml) and acidified with HCl gas. Upon standing, a white solid crystallized which was collected by filtration and vacuum dried to give 3.4 g of 3 - (7,8,9,10 - tetrahydro - 6 - oxo - 6H - dibenzo-[b,d]pyranyl - 3 - oxy) - 1 - cyclopentylamino - 2 - propanol hydrochloride, mp. 224—225°C.

Example 23

Preparation of 3-(7,8,9,10-tetrahydro-6-oxo-6H-dibenzo[b,d]pyranyl-3-oxy)-1-(1H-imidazol-1-yl)-2-propanol hydrochloride

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added imidazole (2.7 g, 0.04 m) and heated to reflux for 3 hours. The solvent was evaporated to a residue, which was purified by chromatography on a Prep 500 HPLC, on silica gel, eluting with chloroform and 5% ammoniated methanol to give the pure amine base as a solid, 6.3 g. This was dissolved in absolute ethanol (100 ml) and ethylacetate (100 ml) and acidified with HCL gas. Upon standing, a white solid crystallized which was collected by filtration and vacuum dried to give 3.4 g of 3 - (7,8,9,10 - tetrahydro - 6 - oxo - 6H - dibenzo[b,d]pyranyl - 3 - oxy) - 1 - (1H - imidazol - 1 - yl) - 2 - propanol hydrochloride, mp. 171—172°C.

Example 24

Preparation of 3-(7,8,9,10-tetrahydro-6-oxo-6H-dibenzo[b,d]pyranyl-3-oxy)-1-(6-oxo-6H-dibenzo[b,d]-pyranyl-3-amino)-2-propanol

To a stirred solution of 3-amino-6-oxo-6H-dibenzo[b,d]pyran (4.77 g, 0.0226 m) in methanol (1.5 L) at reflux, was added 7,8,9,10-tetrahydro-3-(1-propoxy-2,3-oxide)-6-oxo-6H-dibenzo[b,d]pyran (5.8 g, 0.021 m) and the mixture stirred for 5 days, then allowed to cool slowly to ambient temperature. A yellow solid crystallized which was collected by filtration to give 4.8 g crude product. This was dissolved in ethyleneglycol monomethylether (400 ml) at reflux, decolorized with charcoal, hot filtered and the solvent volume reduced to 200 ml. Upon standing, a yellow solid crystallized, which was collected by filtration, washed with methanol and vacuum dried to give 3.0 g of 3 - (7,8,9,10 - tetrahydro - 6 - oxo - 6H - dibenzo[b,d]-pyranyl - 3 - oxy) - 1 - (6 - oxo - 6H - dibenzo[b,d]pyranyl - 3 - amino) - 2 - propanol, mp. 209—210°C.

Example 25

Preparation of 3-(7,8,9,10-Tetrahydro-6-oxo-6H-dibenzo[b,d]pyranyl-3-oxy)-1-amino-2-propanol hydrochloride

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added anhydrous ammonia (25 mls) and the mixture stirred 24 hrs. The solvent was evaporated to a residue, 13.2 g, which was purified by chromatography on a Prep 500 HPLC, on silica gel, eluting with chloroform and 10% ammoniated methanol, to give 4.2 g of an oil. This was dissolved in absolute ethanol (100 ml) and acidified with HCl gas. Upon standing, a white solid crystallized which was collected by filtration and vacuum dried to give 2.4 g of 3 - (7,8,9,10 - tetrahydro - 6 - oxo - 6H - dibenzo[b,d]pyranyl - 3 - oxy) - 1 - (amino) - 2 - propanol hydrochloride, mp 249—250°C.

Example 26

Preparation of N,N-di[3-(7,8,9,10-tetrahydro-6-oxo-6H-dibenzo[b,d]pyranyl-3-oxy)-2-hydroxy propyl]amine hydrochloride

To a stirred solution of 7,8,9,10-tetrahydro-3-(1-propoxy-2,3-oxide)-6-oxo-6H-dibenzo[b,d]pyran (10.0 g, 0.036 m) in methanol (250 ml) and chloroform (50 ml) under an atmosphere of nitrogen was added anhydrous ammonia (25 ml) and the mixture stirred 24 hrs. The solvent was evaporated to a residue, 13.2 g, which was purified by chromatography on a Prep 500 HPLC, on silica gel eluting with chloroform and 10% ammoniated methanol, to give 2.3 g of an oil. This was dissolved in a mixture of methanol (25 ml), water (10 ml), and anhydrous ether (20 ml), and acidified with HCl gas. Upon standing, a white solid crystallized which was collected by filtration and vacuum dried to give 1.2 g of N,N - di[3 - 7,8,9,10 - tetrahydro - 6 - oxo - 6H - dibenzo[b,d]pyranyl - 3 - oxy) - 2 - hydroxy propyl]amine hydrochloride, mp 218—219°C.

The compounds of this invention possess useful antihypoxia activity, that is, they extend the lifetime of animals exposed to a hypoxic environment. This activity is conveniently measured in mice. Groups of mice are tested at various times after the intraperitoneal administration of graded doses of the test compound. The animals' survival time in a hypoxic environment (96% nitrogen and 4% oxygen is recorded. A statistical comparison (Wilcoxon rank sum) is made between coincident vehicle treated animals and the

experimental group. The dose-response, time course and minimum active dose (MAD) for a compound are obtained. Other modes of administration can also be used.

**Claims for the Contracting States: BE CH DE FR GB IT LI LU NL SE**

1. A compound having the formula:

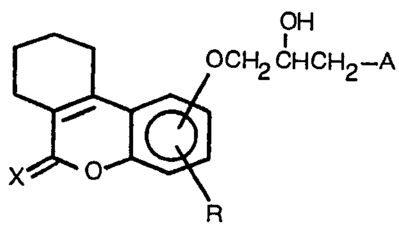

where X is oxygen or dialkyl in which each alkyl group contains one to seven carbons; R is hydrogen or alkyl containing one to seven carbons; and A is

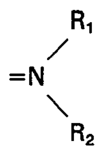

and where $R_1$ and $R_2$ may independently be hydrogen, $C_1$—$C_7$ alkyl, $C_3$—$C_7$ cycloalkyl, aryl, heteroaryl, $C_7$—$C_9$ aralkyl, aryloxyalkyl, $C_2$—$C_6$ alkanol, or where $R_1$ and $R_2$ taken together comprise a heterocyclic ring containing 1—3 heteroatoms and 2—7 carbon atoms.

2. A compound having the formula:

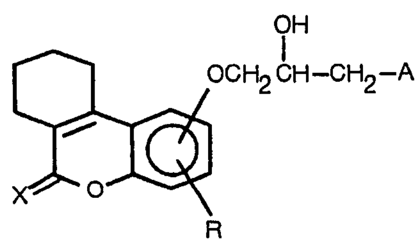

where X is oxygen or dimethyl; R is hydrogen or methyl; and A is

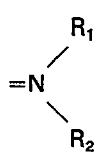

and where $R_1$ and $R_2$ may independently be hydrogen, $C_1$—$C_7$ alkyl, $C_3$—$C_7$ cycloalkyl, aryl, heteroaryl, $C_7$—$C_9$ aralkyl, aryloxyalkyl, $C_2$—$C_6$ alkanol, or where $R_1$ and $R_2$ taken together comprise a heterocyclic ring containing 1—3 heteroatoms and 2—7 carbon atoms.

3. A compound having the formula:

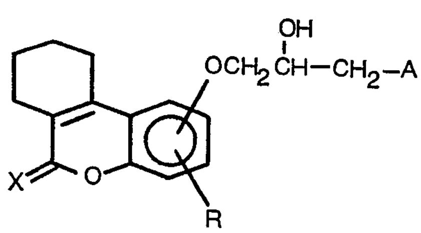

where X is oxygen or dimethyl; R is hydrogen or methyl, and

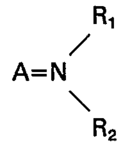

where $R_1$ and $R_2$ may independently be hydrogen, $C_1$—$C_7$ alkyl, $C_3$—$C_7$ cycloalkyl, aryl, heteroaryl, $C_7$—$C_9$ aralkyl, aryloxyalkyl, $C_2$—$C_6$ alkanol, or where $R_1$ and $R_2$ taken together comprise a heterocyclic ring containing 1—3 heteroatoms and 2—7 carbon atoms.

4. A compound having the formula:

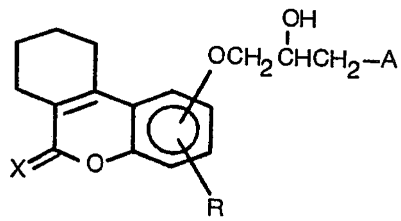

where X is oxygen or dimethyl; R is hydrogen or methyl; and A is amino, methylamino, dimethylamino, ethylamino, dimethylamino, n-propylamino, isopropylamino, cyclopropylamino, t-butylamino, ethanolamino, cyclopentylamino, pyrrolidino, piperidino, anilino, benzylamino, imidazo, 1-phenoxy-2-propylamino, 3-amino-6-oxo-6H-dibenzo[b,d]pyranyl, or 2-hydroxy-3-(7,8,9,10-tetrahydro-6-oxo-6H-dibenzo[b,d]-pyranyl-3-oxy)propylamino.

5. The compound in accordance with claim 4 where X is oxygen.

6. An acid addition salt of the compound according to claim 1.

7. The compound 3-(7,8,9,10-tetrahydro-6-oxo-6H-dibenzo[b,d]pyranyl-3-oxy)-1-(1-piperidinyl)-2-propanol hydrochloride.

8. The compound 3-(7,8,9,10-tetrahydro-6-oxo-6H-dibenzo[b,d]pyranyl-3-oxy)-1-(cyclopropylamino)-2-propanol.

9. The compound 3-(7,8,9,10-tetrahydro-6-oxo-6H-dibenzo[b,d]pyranyl-3-oxy)-1-(1-methylethyl)amino-2-propanol.

10. The compound 3-(7,8,9,10-tetrahydro-3-methyl-6-oxo-6H-dibenzo[b,d]pyranyl-1-oxy)-1-(1,1-dimethylethyl)amino-2-propanol.

11. The compound 3-(7,8,9,10-tetrahydro-6-oxo-6H-dibenzo[b,d]pyranyl-3-oxy)-1-(1H-imidazol-1-yl)-2-propanol hydrochloride.

12. A process for preparing the compound of claims 1 to 11 comprising reacting a phenolic pyran having the general formula:

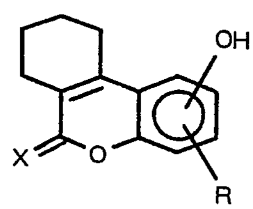

where X and R are as defined in claims 1 to 5 with an alkali metal hydroxide and an excess of an epihalohydrin in an inert solvent and then reacting the resulting epoxide with ammonia or a primary or secondary amine.

**Claims for the Contracting State: AT**

1. A process for preparing a dibenzopyran having the general formula:

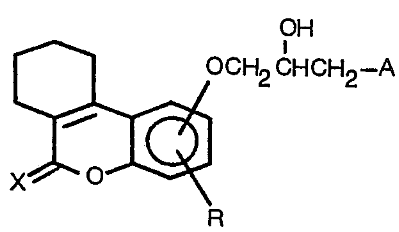

where X is oxygen or dialkyl in which each alkyl group contains one to seven carbons; R is hydrogen or alkyl containing one to seven carbons; and A is

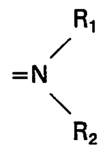

and where $R_1$ and $R_2$ may independently be hydrogen, $C_1$—$C_7$ alkyl, $C_3$—$C_7$ cycloalkyl, aryl, heteroaryl, $C_7$—$C_9$ aralkyl, aryloxyalkyl, $C_2$—$C_6$ alkanol, or where $R_1$ and $R_2$ taken together comprise a heterocyclic ring containing 1—3 heteroatoms and 2—7 carbon atoms comprising reacting a phenolic pyran having the general formula:

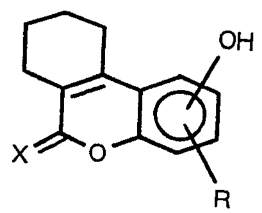

where X and R are as defined above with an alkali metal hydroxide and an excess of an epihalohydrin in an inert solvent and then reacting the resulting epoxide with ammonia or a primary or secondary amine and optionally converting the obtained compound in a pharmaceutically acceptable acid addition salt.

2. The process of claim 1 for the production of a compound having the formula:

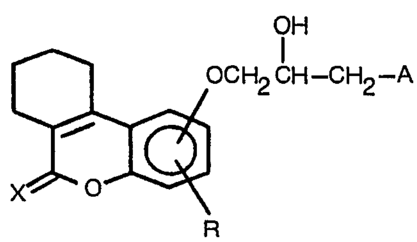

where X is oxygen or dimethyl; R is hydrogen or methyl; and A is

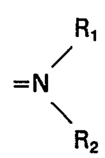

and where $R_1$ and $R_2$ may independently be hydrogen, $C_1$—$C_7$ alkyl, $C_3$—$C_7$ cycloalkyl, aryl, heteroaryl, $C_7$—$C_9$ aralkyl, aryloxyalkyl, $C_2$—$C_6$ alkanol, or where $R_1$ and $R_2$ taken together comprise a heterocyclic ring containing 1—3 heteroatoms and 2—7 carbon atoms.

3. The process of claim 1 for the production of a compound having the formula:

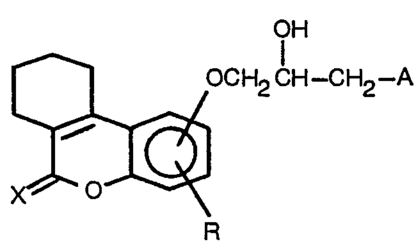

where X is oxygen or dimethyl; R is hydrogen or methyl, and

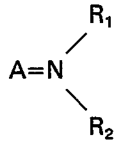

where $R_1$ and $R_2$ may independently be hydrogen, $C_1$—$C_7$ alkyl, $C_3$—$C_7$ cycloalkyl, aryl, heteroaryl, $C_7$—$C_9$ aralkyl, aryloxyalkyl, $C_2$—$C_6$ alkanol, or where $R_1$ and $R_2$ taken together comprise a cyclic or heterocyclic ring containing 0—3 heteroatoms and 2—7 carbon atoms.

4. The process of claim 1 for the production of a compound having the formula:

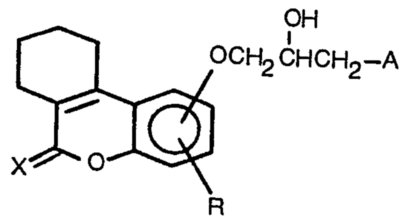

where X is oxygen or dimethyl; R is hydrogen or methyl; and A is amino, methylamino, dimethylamino, ethylamino, dimethylamino, n-propylamino, isopropylamino, cyclopropylamino, t-butylamino, ethanolamino, cyclopentylamino, pyrrolidino, piperidino, analino, benzylamino, imidazo, 1-phenoxy-2-propylamino, 3-amino-6-oxo-6H-dibenzo[b,d]pyranyl, or 2-hydroxy-3-(7,8,9,10-tetrahydro-6-oxo-6H-dibenzo[b,d]-pyranyl-3-oxy)propylamino.

5. The process of claim 1 for the production of a compound in accordance with claim 4 where X is oxygen.

6. The process of claim 1 for the production of an acid addition salt of the compound according to claim 1.

7. The process of claim 1 for the production of the compound 3-(7,8,9,10-tetrahydro-6-oxo-6H-dibenzo-[b,d]pyranyl-3-oxy)-1-(1-piperidinyl)-2-propanol hydrochloride.

8. The process of claim 1 for the production of the compound 3-(7,8,9,10-tetrahydro-6-oxo-6H-dibenzo-[b,d]pyranyl-3-oxy)-1-(cyclopropylamino)-2-propanol.

9. The process of claim 1 for the production of the compound 3-(7,8,9,10-tetrahydro-6-oxo-6H-dibenzo-[b,d]pyranyl-3-oxy)-1-(1-methylethyl)amino-2-propanol.

10. The process of claim 1 for the production of the compound 3-(7,8,9,10-tetrahydro-3-methyl-6-oxo-6H-dibenzo[b,d]pyranyl-1-oxy)-1-(1,1-dimethylethyl)amino-2-propanol.

11. The process of claim 1 for the production of the compound 3-(7,8,9,10-tetrahydro-6-oxo-6H-dibenzo[b,d]pyranyl-3-oxy)-1-(1H-imidazol-1-yl)-2-propanol hydrochloride.

**Patentansprüche für die Vertragsstaaten: BE CH DE FR GB IT LI LU NL SE**

1. Verbindung mit der Formel:

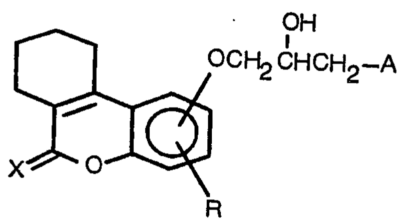

worin X für Sauerstoff oder Dialkyl steht, wobei jede Alkylgruppe 1 bis 7 Kohlenstoffatome enthält, R für Wasserstoff oder Alkyl mit 1 bis 7 Kohlenstoffatomen steht und A für

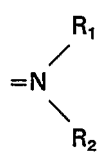

steht und worin $R_1$ und $R_2$ unabhängig voneinander Wasserstoff, $C_1$—$C_7$-Alkyl, $C_3$—$C_7$-Cycloalkyl, Aryl, Heteroaryl, $C_7$—$C_9$-Aralkyl, Aryloxyalkyl, $C_2$—$C_6$-Alkanol sein können oder worin $R_1$ und $R_2$ miteinander

genommen einen heterocyclischen Ring darstellen, der 1 bis 3 Heteroatome und 2 bis 7 Kohlenstoffatome enthält.

2. Verbindung mit der Formel:

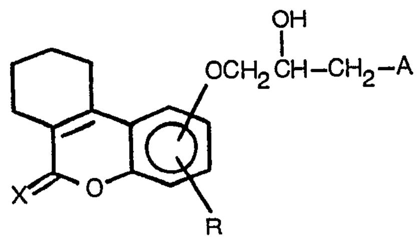

worin X für Sauerstoff oder Dimethyl steht, R für Wasserstoff oder Methyl steht und A für

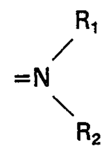

steht und worin $R_1$ und $R_2$ unabhängig voneinander Wasserstoff, $C_1$—$C_7$-Alkyl, $C_3$—$C_7$-Cycloalkyl, Aryl, Heteroaryl, $C_7$—$C_9$-Aralkyl, Aryloxyalkyl, $C_2$—$C_6$-Alkanol sein können oder worin $R_1$ und $R_2$ miteinander genommen einen heterocyclischen Ring darstellen, der 1 bis 3 Heteroatome und 2 bis 7 Kohlenstoffatome enthält.

3. Verbindung mit der Formel:

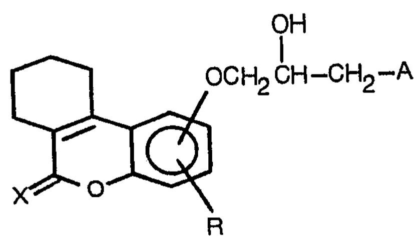

worin X für Sauerstoff oder Dimethyl steht, R für Wasserstoff oder Methyl steht und

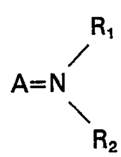

worin $R_1$ und $R_2$ unabhängig voneinander Wasserstoff, $C_1$—$C_7$-Alkyl, $C_3$—$C_7$-Cycloalkyl, Aryl, Heteroaryl, $C_7$—$C_9$-Aralkyl, Aryloxyalkyl, $C_2$—$C_6$-Alkanol sein können oder worin $R_1$ und $R_2$ miteinander genommen einen heterocyclischen Ring darstellen, der 1 bis 3 Heteroatome und 2 bis 7 Kohlenstoffatome enthält.

4. Verbindung mit der Formel:

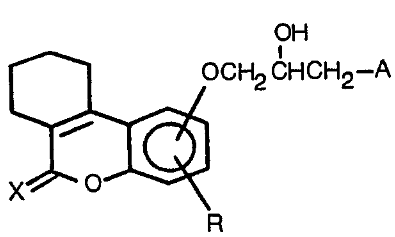

worin X für Sauerstoff oder Dimethyl steht, R für Wasserstoff oder Methyl steht und A für Amino, Methylamino, Dimethylamino, Ethylamino, Dimethylamino, n-Propylamino, Isopropylamino, Cyclopropylamino, t-Butylamino, Ethanolamino, Cyclopentylamino, Pyrrolidino, Piperidino, Anilino, Benzylamino, Imidazo, 1-Phenoxy-2-propylamino, 3-Amino-6-oxo-6H-dibenzo[b,d]pyranyl oder 2-Hydroxy-3-(7,8,9,10-tetrahydro-6-oxo-6H-dibenzo[b,d]pyranyl-3-oxy)propylamino steht.

5. Verbindung nach Anspruch 4, dadurch gekennzeichnet, daß X für Sauerstoff steht.

6. Saüreadditionssalz der Verbindung nach Anspruch 1.

7. Die Verbindung 3-(7,8,9,10-Tetrahydro-6-oxo-6H-dibenzo[b,d]pyranyl-3-oxy)-1-(1-piperidinyl)-2-propanol-hydrochlorid.

8. Die Verbindung 3-(7,8,9,10-Tetrahydro-6-oxo-6H-dibenzo[b,d]pyranyl-3-oxy)-1-(cyclopropylamino)-2-propanol.

9. Die Verbindung 3-(7,8,9,10-Tetrahydro-6-oxo-6H-dibenzo[b,d]pyranyl-3-oxy)-1-(1-methylethyl)-amino-2-propanol.

10. Die Verbindung 3-(7,8,9,10-Tetrahydro-3-methyl-6-oxo-6H-dibenzo[b,d]pyranyl-3-oxy)-1-(1,1-dimethylethyl)amino-2-propanol.

11. Die Verbindung 3-(7,8,9,10-Tetrahydro-6-oxo-6H-dibenzo[b,d]pyranyl-3-oxy)-1-(1H-imidazol-1-yl)-2-propanol-hydrochlorid.

12. Verfahren zur Herstellung der Verbindung nach den Ansprüchen 1 bis 11, dadurch gekennzeichnet, daß man ein phenolisches Pyran der allgemeinen Formel:

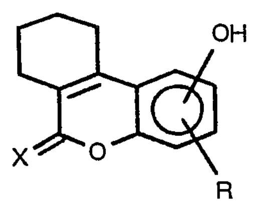

worin X und R wie in den Ansprüchen 1 bis 5 definiert sind, mit einem Alkalimetallhydroxid und einem Überschuß eines Epihalogenhydrins in einem inerten Lösungsmittel umsetzt und sodann das resultierende Epoxid mit Ammoniak oder einem primären oder sekundären Amin umsetzt.

**Patentansprüche für den Vertragsstaat: AT**

1. Verfahren zur Herstellung eines Dibenzopyrans mit der allgemeinen Formel:

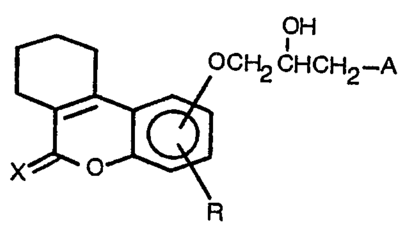

worin X für Sauerstoff oder Dialkyl steht, wobei jede Alkylgruppe 1 bis 7 Kohlenstoffatome enthält, R für Wasserstoff oder Alkyl mit 1 bis 7 Kohlenstoffatomen steht und A für

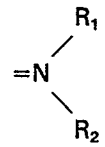

steht und worin $R_1$ und $R_2$ unabhängig voneinander Wasserstoff, $C_1$—$C_7$-Alkyl, $C_3$—$C_7$-Cycloalkyl, Aryl, Heteroaryl, $C_7$—$C_9$-Aralkyl, Aryloxyalkyl, $C_2$—$C_6$-Alkanol sein können oder worin $R_1$ und $R_2$ miteinander genommen einen heterocyclischen Ring darstellen, der 1 bis 3 Heteroatome und 2 bis 7 Kohlenstoffatome enthält, dadurch gekennzeichnet, daß man ein phenolisches Pyran der allgemeinen Formel:

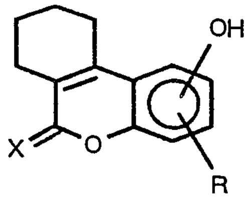

worin X und R wie oben definiert sind, mit einem Alkalimetallhydroxid und einem Überschuß eines Epihalogenhydrins in einem inerten Lösungsmittel umsetzt und sodann das resultierende Epoxid mit Ammoniak oder einem primären oder sekundären Amin umsetzt und gegebenenfalls die so erhaltene Verbindung in ein pharmazeutisch annehmbares Säureadditionssalz umwandelt.

2. Verfahren nach Anspruch 1 zur Herstellung einer Verbindung mit der Formel:

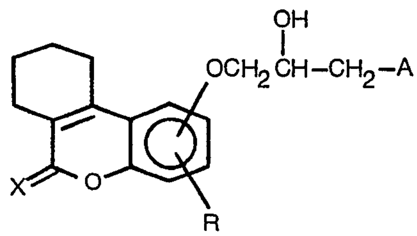

worin X für Sauerstoff oder Dimethyl steht, R für Wasserstoff oder Methyl steht und A für

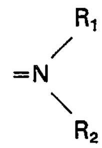

steht und worin $R_1$ und $R_2$ unabhängig voneinander Wasserstoff, $C_1$—$C_7$-Alkyl, $C_3$—$C_7$-Cycloalkyl, Aryl, Heteroaryl, $C_7$—$C_9$-Aralkyl, Aryloxyalkyl, $C_2$—$C_6$-Alkanol sein können oder worin $R_1$ und $R_2$ miteinander genommen einen heterocyclischen Ring darstellen, der 1 bis 3 Heteroatome und 2 bis 7 Kohlenstoffatome enthält.

3. Verfahren nach Anspruch 1 zur Herstellung einer Verbindung mit der Formel:

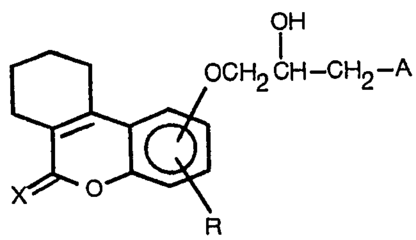

worin X für Sauerstoff oder Dimethyl steht, R für Wasserstoff oder Methyl steht und

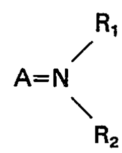

worin $R_1$ und $R_2$ unabhängig voneinander Wasserstoff, $C_1$—$C_7$-Alkyl, $C_3$—$C_7$-Cycloalkyl, Aryl, Heteroaryl, $C_7$—$C_9$-Aralkyl, Aryloxyalkyl, $C_2$—$C_6$-Alkanol sein können oder worin $R_1$ und $R_2$ miteinander genommen einen cyclischen oder heterocyclischen Ring darstellen, der 0 bis 3 Heteroatome und 2 bis 7 Kohlenstoffatome enthält.

4. Verfahren nach Anspruch 1 zur Herstellung einer Verbindung mit der Formel:

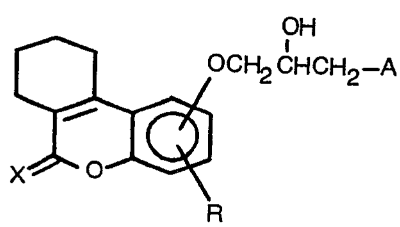

worin X für Sauerstoff oder Dimethyl steht, R für Wasserstoff oder Methyl steht und A für Amino, Methylamino, Dimethylamino, Ethylamino, Dimethylamino, n-Propylamino, Isopropylamino, Cyclopropylamino, t-Butylamino, Ethanolamino, Cyclopentylamino, Pyrroilidino, Piperidino, Anilino, Benzylamino, Imidazo, 1-Phenoxy-2-propylamino, 3-Amino-6-oxo-6H-dibenzo[b,d]pyranyl oder 2-Hydroxy-3-(7,8,9,10-tetrahydro-6-oxo-6H-dibenzo[b,d]pyranyl-3-oxy)propylamino steht.

5. Verfahren nach Anspruch 1 zur Herstellung einer Verbindung nach Anspruch 4, worin X für Sauerstoff steht.

6. Verfahren nach Anspruch 1 zur Herstellung eines Saüreadditionssalzes der Verbindung nach Anspruch 1.

7. Verfahren nach Anspruch 1 zur Herstellung der Verbindung 3-(7,8,9,10-Tetrahydro-6-oxo-6H-dibenzo[b,d]pyranyl-3-oxy)-1-(1-piperidinyl)-2-propanol-hydrochlorid.

8. Verfahren nach Anspruch 1 zur Herstellung der Verbindung 3-(7,8,9,10-Tetrahydro-6-oxo-6H-dibenzo[b,d]pyranyl-3-oxy)-1-(cyclopropylamino)-2-propanol.

9. Verfahren nach Anspruch 1 zur Herstellung der Verbindung 3-(7,8,9,10-Tetrahydro-6-oxo-6H-dibenzo[b,d]pyranyl-3-oxy)-1-(1-methylethyl)amino-2-propanol.

10. Verfahren nach Anspruch 1 zur Herstellung der Verbindung 3-(7,8,9,10-Tetrahydro-3-methyl-6-oxo-6H-dibenzo[b,d]pyranyl-1-oxy)-1-(1,1-dimethylethyl)amino-2-propanol.

11. Verfahren nach Anspruch 1 zur Herstellung der Verbindung 3-(7,8,9,10-Tetrahydro-6-oxo-6H-dibenzo[b,d]pyranyl-3-oxy)-1-(1H-imidazol-1-yl)-2-propanol-hydrochlorid.

**Revendications pour les Etats contractants: BE CH DE FR GB IT LI LU NL SE**

1. Composé de formule:

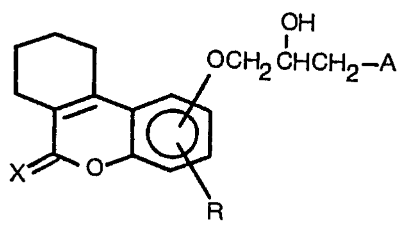

dans laquelle X est l'oxygène ou représente deux groupes alkyle dont chacun contient un à sept atomes de carbone; R est l'hydrogène ou un groupe alkyle contenant un à sept atomes de carbone; et A est un groupe de formule

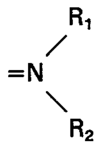

où $R_1$ et $R_2$ peuvent représenter, indépendamment, l'hydrogène, un groupe alkyle en $C_1$ à $C_7$, cycloalkyle en $C_3$ à $C_7$, aryle, hétéro-aryle, aralkyle en $C_7$ à $C_9$, aryloxyalkyle, alcanol en $C_2$ à $C_6$ ou bien $R_1$ et $R_2$ forment conjointement un noyau hétérocyclique contenant 1 à 3 hétéro-atomes et 2 à 7 atomes de carbone.

2. Composé de formule:

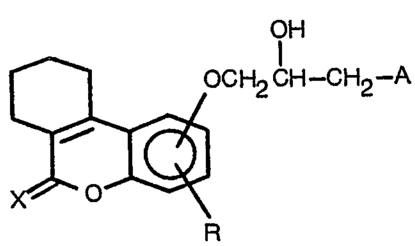

dans laquelle X est l'oxygène ou deux groupes méthyle; R est l'hydrogène ou un groupe méthyle; et A répond à la formule

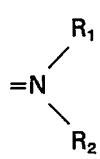

dans laquelle $R_1$ et $R_2$ peuvent représenter, indépendamment, l'hydrogène, un groupe alkyle en $C_1$ à $C_7$, cycloalkyle en $C_3$ à $C_7$, aryle, hétéro-aryle, aralkyle en $C_7$ à $C_9$, aryloxyalkyle, alcanol en $C_2$ à $C_6$, ou bien $R_1$ et $R_2$ forment conjointement un noyau hétérocyclique contenant 1 à 3 hétéro-atomes et 2 à 7 atomes de carbone.

3. Composé de formule:

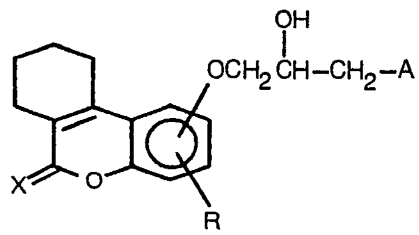

dans laquelle X est l'oxygène ou deux groupes méthyle; R est l'hydrogène ou un groupe méthyle et A est un groupe de formule

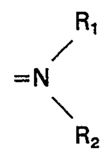

dans laquelle $R_1$ et $R_2$ peuvent représenter, indépendamment, l'hydrogène, un groupe alkyle en $C_1$ à $C_7$, cycloalkyle en $C_3$ à $C_7$, aryle, hétéro-aryle, aralkyle en $C_7$ à $C_9$, aryloxyalkyle, alcanol en $C_2$ à $C_6$, ou bien $R_1$ et $R_2$ forment conjointement un noyau hétérocyclique contenant 1 à 3 hétéro-atomes et 2 à 7 atomes de carbone.

4. Composé de formule:

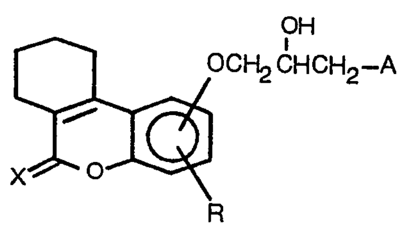

dans laquelle X est l'oxygène ou deux groupes méthyle; R est l'hydrogène ou un groupe méthyle; et A est un groupe amino, méthylamino, diméthylamino, éthylamino, diéthylamino, n-propylamino, isopropylamino, cyclopropylamino, tertio-butylamino; éthanolamino, cyclopentylamino, pyrrolidino, pipéridino, anilino, benzylamino, imidazo, 1-phénoxy-2-propylamino, 3-amino-6-oxo-6H-dibenzo[b,d]pyrannyl ou 2-hydroxy-3-(7,8,9,10-tétrahydro-6-oxo-6H-dibenzo[b,d]pyranyl-3-oxy)propylamino.

5. Composé suivant la revendication 4, dans lequel X est l'oxygène.

6. Un sel d'addition d'acide du composé suivant la revendication 1.

7. Le chlorhydrate de 3-(7,8,9,10-tétrahydro-6-oxo-6H-dibenzo[b,d]pyrannyl-3-oxy)-1-(1-pipéridinyl)-2-propanol.

8. Le 3-(7,8,9,10-tétrahydro-6-oxo-6H-dibenzo[b,d]pyrannyl-3-oxy)-1-(cyclopropylamino)-2-propanol.

9. Le 3-(7,8,9,10-tétrahydro-6-oxo-6H-dibenzo[b,d]pyrannyl-3-oxy)-1-(1-méthyléthyl)amino-2-propanol.

10. Le 3-(7,8,9,10-tétrahydro-3-méthyl-6-oxo-6H-dibenzo[b,d]pyrannyl-1-oxy)-1-(1,1-diméthyléthyl)-amino-2-propanol.

11. Le chlorhydrate de 3-(7,8,9,10-tétrahydro-6-oxo-6H-dibenzo[b,d]pyrannyl-3-oxy)-1-(1H-imidazole-1-yl)-2-propanol.

12. Procédé de préparation du composé suivant les revendications 1 à 11, qui consiste à faire réagir un pyranne phénolique répondant à la formule générale:

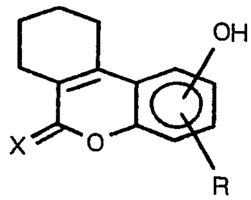

dans laquelle X et R sont tels que définis dans les revendications 1 à 5, avec un hydroxyde de métal alcalin et un excès d'une épihalogénhydrine dans un solvant inerte, puis à faire réagir l'époxyde résultant avec l'ammoniac ou avec une amine primaire ou secondaire.

**Revendications pour l'Etat contractant: AT**

1. Procédé de préparation d'un dibenzopyranne de formule générale:

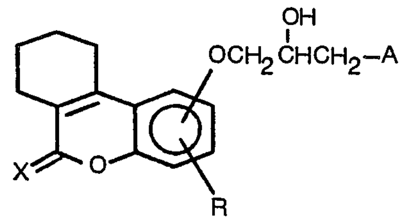

dans laquelle X est l'oxygène ou deux groupes alkyle conenant chacun un à sept atomes de carbone; R est l'hydrogène ou un groupe alkyle contenant un à sept atomes de carbone; et A est un groupe de formule

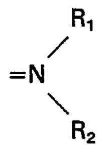

dans laquelle $R_1$ et $R_2$ peuvent représenter, indépendamment, l'hydrogène, un groupe alkyle en $C_1$ à $C_7$, cycloalkyle en $C_3$ à $C_7$, aryle, hétéro-aryle, aralkyle en $C_7$ à $C_9$, aryloxyalkyle, alcanol en $C_2$ à $C_6$ ou bien $R_1$ et $R_2$ forment conjointement un noyau hétérocyclique contenant 1 à 3 hétéro-atomes et 2 à 7 atomes de carbone, qui consiste à faire réagir un pyranne phénolique de formule générale:

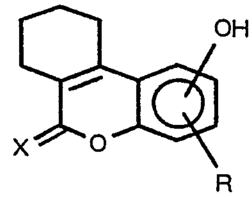

dans laquelle X et R sont tels que définis ci-dessus, avec un hydroxyde de métal alcalin et un excès d'une épihalogénhydrine dans un solvant inerte, puis à faire réagir l'époxyde résultant avec l'ammoniac ou une amine primaire ou secondaire et, à titre facultatif, à convertir le composé obtenu en un sel d'addition d'acide pharmaceutiquement acceptable.

2. Procédé suivant la revendication 1 pour la production d'un composé répondant à la formule:

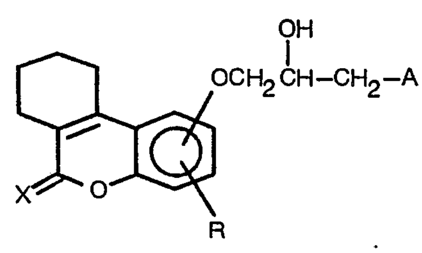

dans laquelle X représente l'oxygène ou deux groupes méthyle; R est l'oxygène ou un groupe méthyle; et A est un groupe de formule

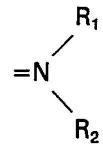

dans laquelle $R_1$ et $R_2$ peuvent représenter, indépendamment, l'hydrogène, un groupe alkyle en $C_1$ à $C_7$, cycloalkyle en $C_3$ à $C_7$, aryle, hétéro-aryle, aralkyle en $C_7$ à $C_9$, aryloxyalkyle, alcanol en $C_2$ à $C_6$, ou bien $R_1$ et $R_2$ forment conjointement un noyau hétérocyclique contenant 1 à 3 hétéro-atomes et 2 à 7 atomes de carbone.

3. Procédé suivant la revendication 1, pour la production d'un composé de formule:

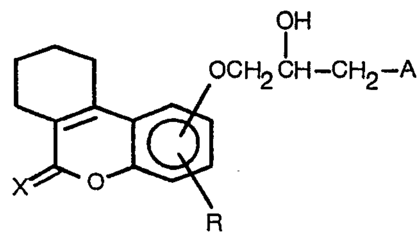

dans laquelle X est l'oxygène ou deux groupes méthyle; R est l'hydrogène ou un groupe méthyle et A répond à la formule

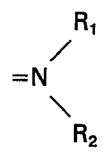

dans laquelle $R_1$ et $R_2$ peuvent représenter, indépendamment, l'hydrogène, un groupe alkyle en $C_1$ à $C_7$, cycloalkyle en $C_3$ à $C_7$, aryle, hétéro-aryle, aralkyle en $C_7$ à $C_9$, aryloxyalkyle, alcanol en $C_2$ à $C_6$, ou bien $R_1$ et $R_2$ forment conjointement un noyau cyclique ou hétérocyclique contenant 0 à 3 hétéro-atomes et 2 à 7 atomes de carbone.

4. Procédé suivant la revendication 1, pour la production d'un composé de formule:

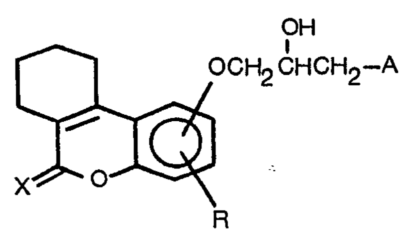

dans laquelle X est l'oxygène ou deux groupes méthyle; R est l'hydrogène ou un groupe méthyle; et A est un groupe amino, méthylamino, diméthylamino, éthylamino, diméthylamino, n-propylamino, isopropylamino, cyclopropylamino, tertio-butylamino; éthanolamino, cyclopentylamino, pyrrolidino, pipéridino, anilino, benzylamino, imidazo, 1-phénoxy-2-propylamino, 3-amino-6-oxo-6H-dibenzo[b,d]pyranyl ou 2-hydroxy-3-(7,8,9,10-tétrahydro-6-oxo-6H-dibenzo[b,d]pyrannyl-3-oxy)propylamino.

5. Procédé suivant la revendication 4, pour la production d'un composé suivant la revendication 4, dans lequel X est l'oxygène.

6. Procédé suivant la revendication 1, pour la production d'un sel d'addition d'acide du composé suivant la revendication 1.

7. Procédé suivant la revendication 1, pour la production du chlorhydrate de 3-(7,8,9,10-tétrahydro-6-oxo-6H-dibenzo[b,d]pyrannyl-3-oxy)-1-(1-pipéridinyl)-2-propanol.

8. Procédé suivant la revendication 1, pour la production du 3-(7,8,9,10-tétrahydro-6-oxo-6H-dibenzo[b,d]pyrannyl-3-oxy)-1-(cyclopropylamino)-2-propanol.

9. Procédé suivant la revendication 1, pour la production du 3-(7,8,9,10-tétrahydro-6-oxo-6H-dibenzo[b,d]pyrannyl-3-oxy)-1-(1-méthyléthyl)amino-2-propanol.

10. Procédé suivant la revendication 1, pour la production du 3-(7,8,9,10-tétrahydro-3-méthyl-6-oxo-6H-dibenzo[b,d]pyrannyl-1-oxy)-1-(1,1-diméthyléthyl)amino-2-propanol.

11. Procédé suivant la revendication 1, pour la production du chlorhydrate de 3-(7,8,9,10-tétrahydro-6-oxo-6H-dibenzo[b,d]pyrannyl-3-oxy)-1-(1H-imidazole-1-yl)-2-propanol.